# EUROPEAN PATENT APPLICATION

(11) **EP 2 081 012 A1**
(43) Date of publication of application: **22.07.2009**
(21) Application number: 09150728.5
(22) Date of filing: 16.01.2009
(51) Int. Cl.: G01N 21/35, G01N 21/88, G01N 33/02

(54) **Method of inspecting food and inspection apparatus implementing the same**

(30) Priority: 18.01.2008 JP 2008009483
(71) Applicant: Sumitomo Electric Industries, Ltd., Osaka-shi, Osaka 541-0041 (JP)
(72) Inventor: Tanaka, Masato, Yokohama-shi Kanagawa (JP); Okuno, Toshiaki, Yokohama-shi Kanagawa (JP); Shimazu, Takayuki, Kanagawa (JP); Katayama, Makoto, Yokohama-shi Kanagawa (JP)
(74) Representative: HOFFMANN EITLE

(57) **Abstract**

A food inspection apparatus of the present invention, which allows throughput improvement, comprises a light source unit 10 for irradiating near-infrared light to an irradiation range including an inspection object 90; a detector unit 20 having a plurality of photodetectors for receiving light such that the light caused in the irradiation range by the irradiation is detected repeatedly at intervals of given time; an analyzer unit 40 for extracting a plurality of features out of the signal groups output by the detector unit 20 according to the detected light intensity; and a display unit 40 for displaying the plurality of features as images.

## Description

### Technical Field

The present invention relates to a method and apparatus for inspecting the quality of a food or the existence/nonexistence of a foreign matter in a food.

### Background Art

In recent years, demand for the safety of food has increased, and therefore, the need for conducting inline analysis about the freshness and quality of the food has been increasing. Inspecting the freshness and quality of food with the naked eye is one way. However, the visual inspection would suffer from differences among individuals, as well as limit in the identification. Moreover, the mixing of foreign matters in foods are serious problems in the production of foods. The mixing of foreign matters occurs in various manners, and when a foreign matter has the same color as the food or is buried in the food, it is difficult to detect with visible light.

The inventions made for the purpose of solving such a problem are disclosed in: for example, Japanese Patent Application Publication Nos. 2004-301690, 2007-010647, 2000-157936, and 2001-099783. At present, a method attracting industrial attention for inspecting the quality of a food and detecting a foreign matter is an analysis using the near infrared light to which food is transparent and which does not suffer from the influence of visible color. Moreover, it is possible to analyze the ingredients of a food by analyzing data obtained wavelengthwise using light of multiple wavelengths.

In the case where a lot of foods are moving on a manufacturing line, for example, it is necessary to process many data at high speed in order to make inline inspection of the foods with near infrared light. Therefore, the improvement in the throughput of food inspection is demanded.

### Disclosure of Invention

Problem to be solved by the invention

The object of the present invention is to provide a food inspection method and apparatus which allow the improvement of throughput. Means for solving the problem to be solved

In order to achieve the object, a method for detecting the existence/nonexistence of a foreign matter in foods or inspecting the quality of foods is provided, wherein the method includes: a step of irradiating near-infrared light to an irradiation range including an inspection object; a step of receiving light with a detector unit including a plurality of photodetectors such that light generated within the irradiation range by the irradiation is detected repeatedly at intervals of given time; a step of selecting signal groups for analysis out of the signal groups output by the detector unit according to the detected light intensity; a step of extracting a plurality of features on the basis of the signal groups for analysis; and a step of displaying the plurality of features as images.

In addition, an apparatus for detecting the existence/nonexistence of a foreign matter in foods or inspecting the quality of foods is provided, wherein the apparatus includes: a light source unit for irradiating near-infrared light to an irradiation range including an inspection object; a detector unit including a plurality of photodetectors for receiving light such that the light caused in the irradiation range by the irradiation is detected repeatedly at intervals of given time; an analyzer unit for selecting signal groups for analysis out of the signal groups output by the detector unit according to the detected light intensity, and extracting a plurality of features on the basis of the signal groups for analysis; and a display unit for displaying the plurality of features as images.

### Brief description of the drawings

Figure 1 is a conceptional schematic diagram illustrating an apparatus for inspecting the quality of a food or detecting the existence/nonexistence of a foreign matter in the food.

Figure 2 is a conceptional schematic diagram showing inspection objects, which are being inspected by the inspection apparatus of Fig. 1, and the vicinity thereof.

Figure 3 is a conceptional schematic diagram of another apparatus for inspecting the quality of a food or detecting the existence/nonexistence of a foreign matter in the food.

Figure 4 is a conceptional schematic diagram showing groups of signals which are output from the detector unit and put into the analyzer unit in the inspection apparatuses in Figs. 1 and 3.

Figures 5A and 5B are conceptional schematic diagrams each showing a manner of culling the data from the groups of signals shown in Fig. 4: Fig. 5A shows an example in which the culling ratio of the portions representing inspection objects and the culling ratio of the background portion are equal; and Fig. 5B shows an example in which the culling ratio of the portions representing inspection objects is less than the culling ratio of the background portion.

Figure 6A is a conceptional schematic diagram for explaining a method of selecting a specific data out of the groups of signals shown in Fig. 4, and Fig. 6B is a conceptional schematic diagram for explaining a method of analyzing the data thus selected.

Figure 7 is a conceptional schematic diagram for explaining a manner of integrating the groups of signals shown in Fig. 4.

### Detailed explanation of the invention

Hereinafter, preferred embodiments of the present invention will be described in reference to the accompanying drawings. The drawings are provided for explaining the embodiments and are not intended to limit the scope of the invention. In the drawings, an identical mark represents the same element so that the repetition of explanation may be omitted. The dimensional ratios in the drawings are not always exact.

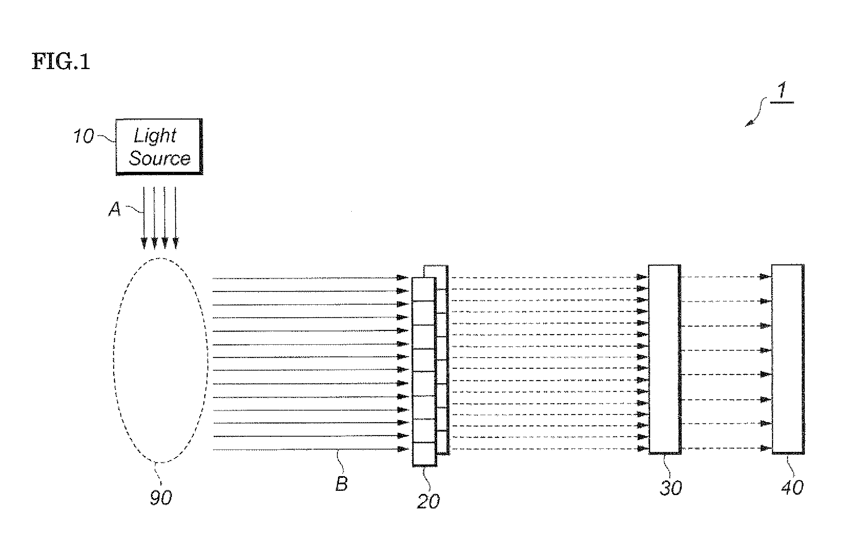

Figure 1 is a conceptional schematic diagram illustrating an apparatus 1 for inspecting the quality of a food or detecting the existence/nonexistence of a foreign matter in the food. The inspection apparatus 1, which is an apparatus for detecting the existence/nonexistence of a foreign matter or inspecting the quality of a food as an inspection object 90, comprises a light source unit 10, a detector unit 20, an analyzer unit 30, and a display unit 40.

The food as the inspection object 90 includes a processed product as well as a material being handled in a manufacturing process. In addition, there may be a case where the inspection object 90 includes a foreign matter or a contaminant besides the food, and the examples of the foreign matter and contaminant includes a hair, a fiber, a remainder of a material (e.g., a skin of a plant, a seed, a bone of an animal, etc.), and an insect. In some cases, the inspection object 90 is contained in a container. It is preferable that the inspection object 90 be moving on a conveyor or in the air during the inspection.

The light source unit 10 irradiates light A in the near-infrared region to an area in which the inspection object 90 lies. The light A includes at least a wavelength component within the wavelength range of 900 nm to 2500 nm, and besides it may also contain a wavelength component outside the wavelength range of 900 nm to 2500 nm.

The detector unit 20 including a plurality of photodetectors receives light by detecting, repeatedly at intervals of a given time, the spatial distribution of light B that is caused in the irradiation range of the light A when the irradiated light A is transmitted through, reflected at, or scattered from the inspection object 90. The detector unit 20 is constituted by arranging a plurality of photodetectors consisting of semiconductors such as InGaAs, Mercury Cadmium Telluride (MCT), PbSe, InSb, etc. which can detect light in the near-infrared region.

The analyzer unit 30 extracts a plurality of features from the detector unit 20 by analyzing signal groups output according to the detected light intensity. For example, the processing modes of output from the analyzer unit 30 are as follows: (Mode 1) the output is made in a color tone converted from the intensity of light detected by each photodetector of the detector unit 20; (Mode 2) the output shows whether the detected light intensity of each photodetector of the detector unit 20 meets a pre-determined condition (e.g., binary value display); and (Mode 3) the detected light intensities of the photodetectors of the detector unit 20 are shown by classifying according to pre-determined conditions (e.g., grouping of the linked pixels). Also, it is desirable that prior to these processing, the analyzer unit 30 be subjected to elimination of noise and the improvement of contrast by image processing such as median filtering and Laplacian filtering to make most of two-dimensional information.

The display unit 40 displays in an image mode the features obtained as a result of the extraction made by the analyzer unit 30. Here, the display mode of the display unit 40 may be a mode to display a result of each pixel, or may be a mode to display the number of the pixels that have met the specific pre-determined conditions according to the above-mentioned second or third mode of the analyzer unit 30. Also, when the output is made in a numerical form, the analyzer unit 30 may be designed to give an alarm sound or to send a trigger signal to another equipment. For example, if a trigger signal is sent to a remover, the remover will be able to remove the foreign matter detected as a foreign matter.

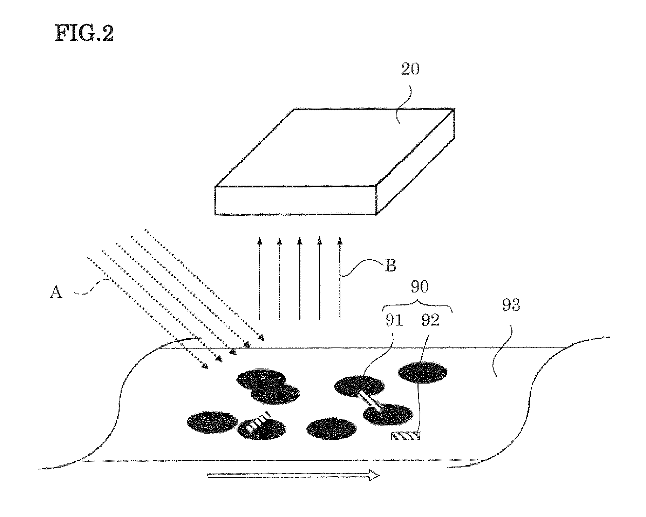

Figure 2 is a conceptional schematic diagram showing inspection objects, which are being inspected by the inspection apparatus 1, and the vicinity thereof. The inspection objects 90 including foods 91 and foreign matters (including contaminants) 92, which are put on a belt conveyor 93, are moving in parallel in a constant direction. Near-infrared light A output from the light source unit 10 is irradiated to the region which includes the inspection objects 90. The light B which arises according to the irradiation in the irradiation range of light A is repeatedly detected at intervals of given time and received by the detector unit 20 having a plurality of photodetectors arranged in an array form.

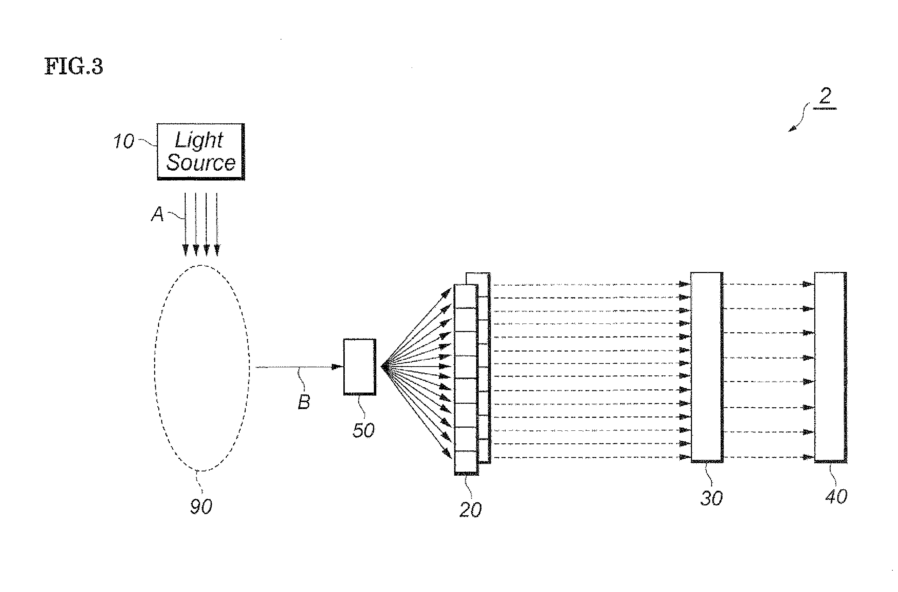

Figure 3 is a conceptional schematic diagram of another apparatus 2 for inspecting the quality of a food or detecting the existence/non-existence of a foreign matter in the food. The inspection apparatus 2, which is an apparatus for inspecting the quality of a food as an inspection object 90 or detecting the existence/nonexistence of a foreign matter in the food, comprises a light source unit 10, a detector unit 20, an analyzer unit 30, a display unit 40, and a spectroscope unit 50.

Light B, which is caused in the irradiation range of the light A by the irradiation of the light A emitted from the light-source unit 10, is separated wavelength-wise by the spectroscope unit 50 which is provided between the inspection object 90 and the detector unit 20. The detector unit 20 receives light having the respective wavelength components separated by the spectroscope unit 50 and outputs signals for showing the spectrum of the light B. The spectrum signals thus output from the detector unit 20 are analyzed by the analyzer unit 30.

In this case, it does not matter whether the light B which is input to the spectroscope 50 has arisen from one region or a plurality of regions of the inspection object 90. In the latter case, with respect to the latter stages after the detector unit 20, the signals may be treated as the two dimensional information consisting of wavelength and position axes.

The output modes of the analyzer unit 30 include a mode in which the strength of a selected wavelength or wavelength band is converted into a color tone for display and a mode in which the result quantified by a calibration curve is shown. It is desirable that prior to these treatments in the analyzer unit 30, the noise be removed and the variation be decreased by performing pre-spectrum processing such as smoothing, baseline correction, or second derivation.

When a large number of inspection objects 90 which move on a line is to be inspected in-line by the inspection apparatus 1 and 2, the analyzer unit 30 must process much data at high speed. Therefore, it is sought to reduce the signal treatment time and to improve the throughput of the food inspection.

When foods as the inspection objects 90 are to be inspected through their images, in some cases an extremely large number of inspection objects 90 having irregular shapes must be inspected while they are placed in a disorderly manner. Also, there may be a case where processed products standing in a row on a manufacturing line must be inspected simultaneously for a plurality of manufacturing lines. Furthermore, in some case, as in the case of the inspection objects 90 having a slender shape, the region that requires a high resolution image with respect to a specific direction only is continuous. In any of those cases, if all regions are inspected in a uniform manner, it will result in redundant inspection data. Also, in the case of spectrum inspection of foods, the optical absorption that occurs due to the existence of ingredients of each inspection object 90 depends on a limited band. Consequently, if a continuous spectrum is detected, it will result in including unnecessary data. Therefore, efficient detection operation is performed by analyzing signal groups which the detector unit 20 outputs according to the detected light intensities and by extracting a plurality of features on the basis of the signal groups which are judged to be more significant one of the output signal groups.

A food, that is an inspection object 90, is composed of various substances which are mixed together, and is **characterized in that** the variation of composition due to the position of its existence in the food is relatively small. Therefore, in many cases of food inspection, the intensities of adjacent photodetectors in the detector unit 20 are almost the same. Also, in some cases, it is sufficient if the existence of abnormal quality or a foreign matter is discovered in the food and it is unnecessary to specify the location of the abnormal quality or the foreign matter in detail. Therefore, according to the present invention, it is sought to reduce the processing time by decreasing the volume of data to be processed, taking the characteristics of inspection objects or methods into consideration. In the following, explanation will be given mainly with respect to image modes; however, similar processing is possible with respect to spectrum as well.

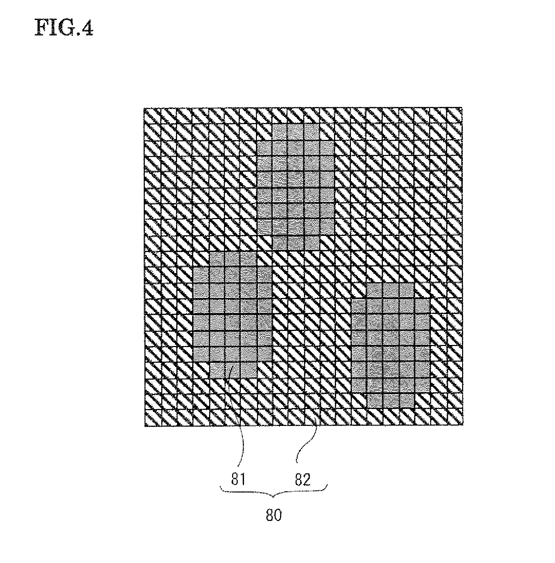

Figure 4 is a conceptional schematic diagram showing groups of signals (image data 80) which are output from the detector unit 20 and put into the analyzer unit 30 in the inspection apparatuses 1 and 2. The grids of the drawing each correspond to the respective photodetectors of the detector unit 20. The image data 80 includes grid portions 81 (light colored parts) showing inspection objects and the background portion 82 (hatched part) showing the part other than the inspection objects.

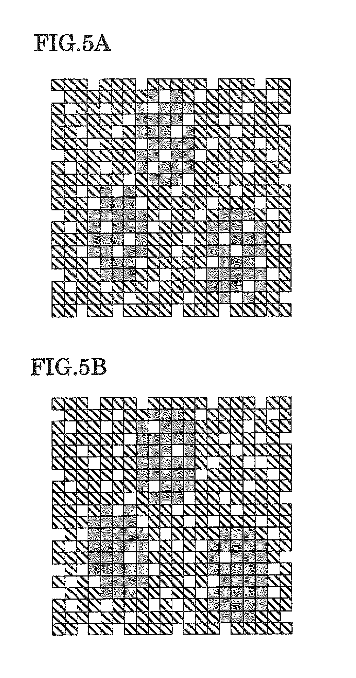

Figures 5A and 5B are conceptional schematic diagrams each showing a manner of culling the data input into the analyzer unit 30: Fig. 5A shows an example in which the culling ratio of the grid portions 81 representing inspection objects and the culling ratio of the background portion 82 are equal; and Fig. 5B shows an example in which the culling ratio of the grid portions 81 representing inspection objects is less than the culling ratio of the background portion 82. In Figs. 5A and 5B, the grid portions 81 representing inspection objects are indicated with a darker color than the background portion 82.

In the analyzer unit 30, the image data 80 output from the detector unit 20 according to the detected light intensity is analyzed after the data has been culled at the ratio determined from the viewpoint of analysis time. Even if some pixels are culled from the image data 80, interpolation can be made with the adjacent pixels so that the number of data may be reduced without degrading the inspection accuracy. The culling may be done in a uniform manner. However, it is possible to increase the culling ratio by changing it according to the detected intensity. The uniform culling does not pose a problem because the approximate positional identification can be made from the order of the data.

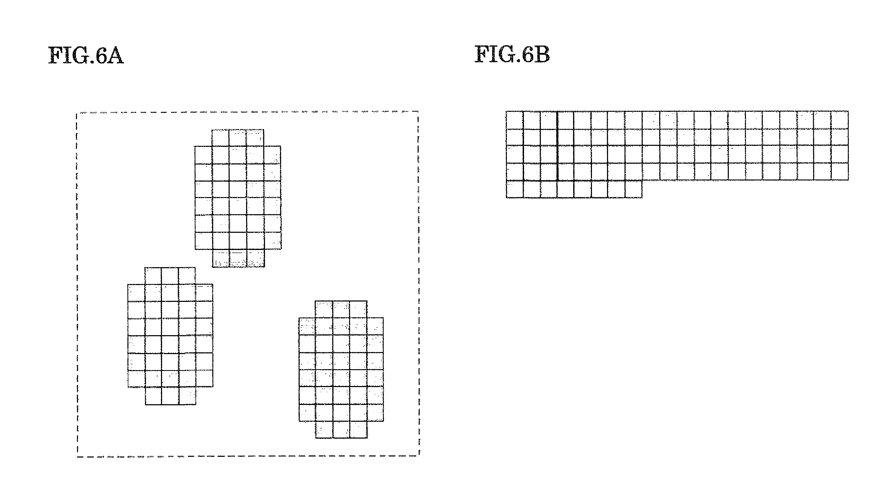

Figure 6A is a conceptional schematic diagram for explaining a method of selecting a specific data out of the image data input into the analyzer unit 30, and Fig. 6B is a conceptional schematic diagram for explaining a method of analyzing the data thus selected. In the case where an inspection object such as a processed product tends to exhibit the detection intensity that falls within a certain scope, it is possible to select pixels whose intensity meets predetermined conditions and analyze those pixels only. In such case, the analyzer unit 30 selects signal groups whose detected light intensities fall within the range previously set, and analyzes them. (In that case, the positional information is lost.) Although this method is unable to identify the position, it is sufficient for the purpose of just detecting abnormalities.

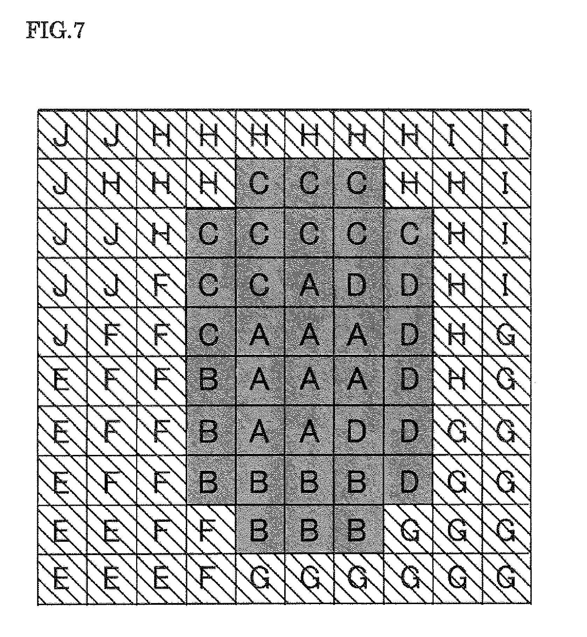

Figure 7 is a conceptional schematic diagram for explaining a manner of integrating the image data input into the analyzer unit 30. In Fig. 7, the portions 81 representing inspection objects are indicated with a darker color than the background portion 82. For analyzing image data output from the detector unit 20 according to the detected light intensity, the analyzer unit 30 first examines the data in terms of the interrelation between the respective pixels, and if the differences in the examination results between adjacent regions are smaller than a predetermined value, then the analyzer unit 30 integrates the relevant signals and thereafter analyzes the image data. In Fig. 7, the results of such integration are indicated with alphabetical letters. In this way, similar pixel data may be put together. For example, cluster analysis may be adopted such that the intensity (or, together with positional information) is a variable relative to each pixel. In this case, positional detection is possible, since positional information is stored in terms of grouping after classification.

The present invention is not limited to the above-described embodiments, and various modifications are possible. In the above embodiments, for example, an explanation was given with respect to culling, selection, and integration for signal groups to form image data; however, the culling, selection, and integration are similarly possible in the case where the signal groups form two-dimensional information (hyper-spectrum) represented with coordinates of wavelength and position.

## Claims

1. An inspection method for detecting the existence/nonexistence of a foreign matter in foods or inspecting the quality of foods, the method comprising:
a step of irradiating near-infrared light to an irradiation range including an inspection object;
a step of receiving light with a detector unit including a plurality of photodetectors such that light generated within the irradiation range by the irradiation is detected repeatedly at intervals of given time;
a step of selecting signal groups for analysis out of the signal groups output by the detector unit according to the detected light intensity;
a step of extracting a plurality of features on the basis of the signal groups for analysis; and
a step of displaying the plurality of features as images.

2. An inspection method according to claim 1, further comprising a step of wavelength-wise separating the light generated within the irradiation range by the irradiation,
wherein light having wavelength components thus separated is received at the step of receiving light.

3. An inspection method according to claim 1,
wherein the spatial distribution of the light caused in the irradiation range by the irradiation is detected at the step of receiving light.

4. An inspection method according to claim 1,
wherein at the step of selecting signal groups, the selection of signal groups for analysis is done by culling, at the ratio determined from the time of analysis, the signal groups output from the detector unit according to detected light intensity.

5. An inspection method according to claim 1,
wherein at the step of selecting signal groups, the selection of signal groups for analysis is done such that the signal groups output from the detector unit according to detected light intensity are limited to the signal groups whose detected light intensities fall within the range previously set.

6. An inspection method according to claim 1,
wherein at the step of selecting signal groups, the selection of signal groups is done by examining the interrelations between the signal groups output by the detector unit according to the detected light intensity, and by integrating the signal groups if their differences in the examination results between adjacent regions are smaller than a predetermined value.

7. An apparatus for detecting the existence/nonexistence of a foreign matter in foods or inspecting the quality of foods, comprising:
a light source unit for irradiating near-infrared light to an irradiation range including an inspection object;
a detector unit including a plurality of photodetectors for receiving light such that the light caused in the irradiation range by the irradiation is detected repeatedly at intervals of given time;
an analyzer unit for selecting signal groups for analysis out of the signal groups output by the detector unit according to the detected light intensity, and extracting a plurality of features on the basis of the signal groups for analysis; and
a display unit for displaying the plurality of features as images.

8. An inspection apparatus according to claim 7, further comprising a spectroscope unit to wavelength-wise separate light having arisen due to the irradiation in the irradiation range,
wherein the detector unit receives light of each wavelength component after such light separation.

9. An inspection apparatus according to claim 7,
wherein the detector unit detects spatial distribution of light caused by the irradiation in the irradiation range.

10. An inspection apparatus according to claim 7,
wherein the analyzer unit selects the signal groups for analysis, culling at the ratio determined from the viewpoint of analysis time, out of the signal groups output from the detector unit according to detected light intensity.

11. An inspection apparatus according to claim 7,
wherein the analyzer unit selects the signal groups for analysis by limiting to the signal groups whose detected light intensities fall within the range previously set.

12. An inspection apparatus according to claim 7,
wherein the signal groups for analysis are selected by the analyzer unit such that the signal groups output by the detector unit according to the detected light intensity are integrated if the differences in their examination results between adjacent regions are smaller than a predetermined value when the interrelations between the signal groups are examined.
